# EUROPÄISCHE PATENTSCHRIFT

(11) **EP 3 394 241 B1**
(45) Veröffentlichungstag und Bekanntmachung des Hinweises auf die Patenterteilung: **25.09.2019**
(21) Anmeldenummer: 16825763.2
(22) Anmeldetag: 22.12.2016
(51) Int. Cl.: C12M 1/34, G01N 21/03

(54) **BEHÄLTER MIT EINER MESSZELLE**
CONTAINER HAVING A MEASURING CELL
RÉCIPIENT PRÉSENTANT UNE CELLULE DE MESURE

(30) Priorität: 23.12.2015 DE 102015122745
(43) Veröffentlichungstag der Anmeldung: 31.10.2018
(73) Patentinhaber: Sartorius Stedim Biotech GmbH, 37079 Göttingen (DE)
(72) Erfinder: HOEHSE, Marek, 37073 Göttingen (DE); GRIMM, Christian, 37308 Heiligenstadt (DE)
(74) Vertreter: Schneider, Peter Christian
(86) Internationale Anmeldenummer: PCT/EP2016/082436
(87) Internationale Veröffentlichungsnummer: WO 2017/109104

(56) Entgegenhaltungen:
- DE-B4-102008 036 934
- US-A1- 2008 032 389
- US-A1- 2015 330 903

## Beschreibung

### Gebiet der Erfindung

Die Erfindung betrifft einen Behälter mit einem in seinen Behälterinnenraum hineinragenden Messzellengehäuse einer optischen Messzelle, wobei das Messzellengehäuse einen Messspalt aufweist, der von zwei einander in einem Abstand gegenüberliegenden Seitenflächen und einer die Seitenflächen verbindenden Verbindungsfläche begrenzt wird, wobei die Seitenflächen jeweils ein optisches Fenster aufweisen, und wobei dem ersten Fenster mindestens eine erste optische Faser und dem zweiten Fenster mindestens eine zweite optische Faser vorgelagert werden kann.

### Stand der Technik

Aus der DE 10 2008 036 934 B4 ist ein Bioreaktor (Behälter) mit einem in seinen Behälterinnenraum hineinragenden Messzellengehäuse einer optischen Messzelle bekannt. Das Messzellengehäuse ist als Teil einer Transmissionssonde ausgebildet, die mit ihrem freien Ende in den Behälterinnenraum des Bioreaktors hineinragt und ein erstes optisches Fenster sowie beabstandet ein zweites optisches Fenster aufweist, die einen Küvetten- oder Messspalt begrenzen, der von dem Probenvolumen des Behälterinnenraumes ausgefüllt ist. Dabei ist dem ersten Fenster eine erste optische Faser und dem zweiten Fenster eine zweite optische Faser vorgelagert. US 2015/330903 A1 beschreibt einen Einweg-Bioreaktor mit einem in seinen Behälterinnenraum hineinragenden Messzellengehäuse einer optischen Messzelle.

Nachteilig bei dem bekannten Bioreaktor (Behälter), der sich grundsätzlich bewährt hat, ist, dass das erste optische Fenster von einem Teilbereich eines Umlenkprismas gebildet wird, was - insbesondere für Einweg-Bioreaktoren (Single-Use-Bioreaktoren) - relativ aufwendig und kostenintensiv ist.

Aus Sterilitätsgründen wird bei Single-Use-Bioreaktoren bzw. Einweg-Bioreaktoren die optische Messzelle eingeschweißt und mit sterilisiert. Für unterschiedliche spektroskopische Techniken müssen daher unterschiedliche Messzellen für jede Spektroskopieart entwickelt, qualifiziert, validiert und eingeschweißt werden.

### Aufgabenstellung

Es ist daher die Aufgabe der vorliegenden Erfindung, einen Behälter, insbesondere zur Verwendung als Einweg-Bioreaktor (Single-Use-Bioreaktor), mit einer kostengünstigen optischen Messzelle für unterschiedliche Spektroskopiearten zu schaffen.

### Darlegung der Erfindung

Diese Aufgabe wird in Verbindung mit den Merkmalen des Oberbegriffs von Anspruch 1 dadurch gelöst, dass das Messzellengehäuse den Fenstern vorgelagerte Aufnahmekanäle zur Aufnahme der jeweils mindestens einen optischen Faser aufweist, dass die Aufnahmekanäle nachträglich von außen her mit den optischen Fasern bestückbar sind, und dass das Messzellengehäuse mit den Fenstern und Aufnahmekanälen fest mit der Wandung des Behälterinnenraumes verbunden ist.

Da die Aufnahmekanäle in Abhängigkeit von der gewählten Spektroskopieart nachträglich mit den mit den optischen Fasern bestückbar sind, kann das Messzellengehäuse kostengünstig in die Behälter eingebaut und universell für optische Messzellen für unterschiedliche Spektroskopiearten genutzt werden.

Der modulare Aufbau erlaubt verschiedene optische Kombinationen ohne den prinzipiellen Aufbau verändern zu müssen. Weiterhin ermöglicht der optische Aufbau der multifunktionalen (Einweg-)Messzelle die Minimierung optischer Komponenten im Einwegteil mit entsprechender Kostenreduktion. Im Gegensatz zu bekannten Sensorköpfen sind die Fasern als Lichtleiter nicht fest in die Sonde integriert sondern können über die Aufnahmekanäle bedarfsabhängig je nach Spektroskopieart in den Einwegteil der optischen Messzelle reversibel eingebracht werden.

Bevorzugte Ausführungsformen sind Gegenstand der abhängigen Ansprüche.

Gemäß einer bevorzugten Ausführungsform der Erfindung sind die Aufnahmekanäle an ihren den Fenstern zugewandten Enden orthogonal zu den Fenstern ausgerichtet.

Beispielsweise durch eine entsprechende Krümmung der Aufnahmekanäle wird eine orthogonale Ausrichtung der Faserenden nach Einführung der Fasern sichergestellt. Dadurch kann beispielsweise auf ein Umlenkprisma oder einen Umlenkspiegel verzichtet werden.

Nach einer weiteren bevorzugten Ausführungsform der Erfindung weist das Messzellengehäuse an seiner Verbindungsfläche ein drittes optisches Fenster auf, wobei dem dritten optischen Fenster ein dritter Aufnahmekanal vorgelagert ist, der nachträglich von außen her mit mindestens einer dritten optischen Faser bestückbar ist.

Durch das dritte Fenster kann dabei Licht aus dem Messspalt zur dritten optischen Faser in einem Winkel von beispielsweise 90° gegenüber der ersten und zweiten Faser geleitet werden. Über die dritte Faser kann das Licht zu einem Detektor geleitet werden.

Grundsätzlich kann in den dritten Aufnahmekanal auch eine abbildende Mehrfachlinsenoptik (wie bei einem Mikroskop) eingebracht werden. Das Einwegteil ist auch dabei nur das dritte Fenster oder eine letzte Linse, alle anderen Optikelemente können reversibel in den dritten Aufnahmekanal eingebracht und wieder entfernt werden. Diese Mehrfachlinsenoptik kann z.B. für die Ramanspektroskopie sowie für die Dunkelfeldmikroskopie verwendet werden.

Die beiden anderen Aufnahmekanäle müssen dabei nicht zwangsläufig ebenfalls verwendet werden, sondern können auch blind bleiben. Der dritte Aufnahmekanal kann, soweit er ein entfernbares drittes Fenster aufweist, grundsätzlich auch für eine Probeentnahme verwendet werden. Dadurch kann sichergestellt werden, dass die entnommene Probe nahezu identisch mit der gerade gemessenen Probenmenge ist. Inhomogenitäten im Behälterinnenraum können sich dadurch nicht auf die zur Kalibrierung entnommenen Proben auswirken. Der dritte Aufnahmekanal kann somit wahlweise der Probeentnahme, der Detektion einer 90° Streuung oder dem Einbringen einer Mehrfachlinsenoptik dienen.

Nach einer weiteren bevorzugten Ausführungsform der Erfindung weisen die Aufnahmekanäle an ihren den Fenstern abgewandten Enden Aufnahmeöffnungen auf, deren Längsachsen parallel zu einander verlaufen.

Durch die parallele Anordnung der Längsachsen können die Fasern durch entsprechende Steck-, Rast- oder Schraubverbindungen mit dem Messzellengehäuse verbunden werden. Die parallelen Längsachsen ermöglichen dabei insbesondere auch eine gemeinsame Steckverbindung.

Nach einer weiteren bevorzugten Ausführungsform bilden die Aufnahmekanäle des Messzellengehäuses eine gemeinsame räumliche Vertiefung in welcher die optischen Fasern durch Halterungen befestigt den Fenstern vorgelagert sind.

Vorteil dieser Anordnung ist die Möglichkeit zur schnelleren und präziseren Ausrichtung der optischen Fasern. Die optischen Fasern können in der räumlichen Vertiefung durch Halterungen so befestigt werden, dass sie den Fenstern vorgelagert sind.

Gemäß einer weiteren bevorzugten Ausführungsform der Erfindung bildet das Messzellengehäuse mit Fenstern und Aufnahmekanälen einen Single-Use-Teil der optischen Messzelle und die optischen Fasern bilden mit verbundenen Lichtquellen und/oder Sensoren und einer Auswerte- und Steuerelektronik einen wiederverwendbaren Teil der optischen Messzelle.

Nach einer weiteren bevorzugten Ausführungsform der Erfindung sind die optischen Fenster als Linsen ausgebildet. Grundsätzlich können die Fenster auch als Filter oder auch als diffus reflektierende Flächen ausgebildet sein.

Gemäß einer weiteren bevorzugten Ausführungsform der Erfindung ist zur Transmission Licht einer Lichtquelle über die mindestens eine erste Faser im ersten Aufnahmekanal durch das erste Fenster in den Messspalt mit einem Probevolumen und weiter über das zweite Fenster und die im zweiten Aufnahmekanal angeordnete mindestens eine zweite Faser zu einem Detektor leitbar. Dabei kann zwischen dem zweiten Fenster oder Linse und der im zweiten Aufnahmekanal angeordneten zweiten Faser mindestens eine Lochblende angeordnet sein. Dabei kann das der Lochblende zugewandte Ende der zweiten Faser beabstandet zur Lochblende angeordnet sein.

Damit wird mit der Vorrichtung relativ einfach und kostengünstig eine Auswertung durch spektroskopische Transmission ermöglicht.

Gemäß einer weiteren bevorzugten Ausführungsform der Erfindung ist zur Reflektion Licht einer Lichtquelle über eine erste Faser im ersten Aufnahmekanal durch das erste Fenster in den Messspalt mit einem Probevolumen leitbar und als vom Probevolumen reflektiertes oder gestreutes Licht zurück über das erste Fenster und eine im ersten Aufnahmekanal angeordnete zweite Faser zu einem Detektor leitbar. Grundsätzlich können statt Fasern auch Faserbündel verwendet werden. Speziell bei einer Reflektionsmessung kann zumindest die Detektion über Faserbündel erfolgen (z.B. ringförmig um Anregungsfaser).

Damit wird mit der Vorrichtung relativ einfach und kostengünstig eine Auswertung durch spektroskopische Reflektion ermöglicht.

Nach einer weiteren bevorzugten Ausführungsform der Erfindung ist zur Transflexion Licht einer Lichtquelle über eine erste Faser im ersten Aufnahmekanal durch das erste Fenster in den Messspalt mit einem Probevolumen und weiter über das zweite Fenster im zweiten Aufnahmekanal auf eine diffus reflektierende Oberfläche leitbar und ist als reflektiertes Licht zurück über das erste Fenster und eine im ersten Aufnahmekanal angeordnete zweite Faser zu einem Detektor leitbar. Dabei kann die reflektierende Oberfläche am freien Ende einer in den zweiten Aufnahmekanal eingeführten Blindfaser (oder bspw. eines Schlauchelementes) angeordnet sein. Grundsätzlich kann auch das zweite Fenster als Reflektor ausgebildet sein.

Damit wird mit der Vorrichtung relativ einfach und kostengünstig eine Auswertung durch spektroskopische Transflexion ermöglicht.

Nach einer weiteren bevorzugten Ausführungsform der Erfindung ist von dem Probevolumen gestreutes Licht, d. h. Licht nach Wechselwirkung mit der Probe, über das dritte optische Fenster und die im dritten Aufnahmekanal angeordnete dritte optische Faser zu einem Detektor leitbar.

Gemäß einer bevorzugten Ausführungsform der Erfindung ist der Behälter als ein Einweg-Bioreaktor oder als ein Einweg-Mischbeutel ausgebildet.

Weitere Merkmale und Vorteile der Erfindung ergeben sich aus der nachfolgenden speziellen Beschreibung und den Zeichnungen.

### Kurzbeschreibung der Zeichnungen

Es zeigen:
- Figur 1:: eine Seitenansicht im Schnitt und Ausriss eines gestrichelt dargestellten Behälters (Einweg-Bioreaktors) mit einem Messzellengehäuse mit Aufnahmekanälen und optischen Fenstern;
- Figur 2:: das Messzellengehäuse von Figur 1 mit einer ersten Faser im ersten Aufnahmekanal und einer zweiten Faser im zweiten Aufnahmekanal;
- Figur 3:: das Messzellengehäuse von Figur 1 mit einer ersten Faser im ersten Aufnahmekanal, einer zweiten Faser im zweiten Aufnahmekanal und einer dritten Faser im dritten Aufnahmekanal;
- Figur 4:: das Messzellengehäuse von Figur 1 mit einer ersten Faser und einer zweiten Faser im ersten Aufnahmekanal;
- Figur 5:: das Messzellengehäuse von Figur 1 mit einer ersten Faser und einer zweiten Faser im ersten Aufnahmekanal und mit einer Blindfaser mit einer diffus reflektierenden Oberfläche im zweiten Aufnahmekanal;
- Figur 6:: eine Seitenansicht im Schnitt und Ausriss eines gestrichelt dargestellten weiteren Behälters (Einweg-Bioreaktors) mit einem Messzellengehäuse mit Aufnahmekanälen und optischen Fenstern;
- Figur 7:: eine räumliche Darstellung eines weiteren Messzellengehäuses dessen Aufnahmekanäle zu einer gemeinsamen Vertiefung zusammengefasst sind;
- Figur 8:: eine Seitenansicht des Messgehäuses von Figur 7 aus Richtung VIII;
- Figur 9:: eine Draufsicht auf das Messgehäuse von Figur 8 aus Richtung IX und
- Figur 10:: eine Seitenansicht des Messgehäuses von Figur 8 mit einem gestrichelt angedeuteten Abdeckteil.

### Beschreibung bevorzugter Ausführungsformen

Ein Behälter 1, beispielsweise ein Einweg-Bioreaktor, besteht im Wesentlichen aus einem Behälterinnenraum 2, einer Wandung 3 und einer optischen Messzelle 4.

Der Behälterinnenraum 2 wird von der Wandung 3 umschlossen. Die Messzelle 4 weist ein Messzellengehäuse 5 auf, das durch die Wandung 3, mit der es fest verbunden ist, in den Behälterinnenraum 2 hineinragt. Behälterinnenraumseitig weist das Messzellengehäuse 5 einen u-förmig ausgeschnittenen Messspalt 6 zur Aufnahme eines zu untersuchenden Probevolumens auf. Der Messspalt 6 wird durch zwei in einem Abstand 7 gegenüberliegende Seitenflächen 8, 9 und einer die Seitenflächen 8, 9 verbindenden Verbindungsfläche 10 begrenzt. Die erste Seitenfläche 8 weist ein erstes optisches Fenster 11, die zweite Seitenfläche 9 weist ein zweites optisches Fenster 12 und die Verbindungsfläche 10 weist ein drittes optisches Fenster 13 auf.

Das Messzellengehäuse 5 weist einen dem ersten Fenster 11 auf seiner dem Messspalt 6 abgewandten Seite vorgelagerten ersten Aufnahmekanal 14 zur Aufnahme mindestens einer ersten optischen Faser 15 auf. Entsprechend weist das Messzellengehäuse 5 einen dem zweiten Fenster 12 auf seiner dem Messspalt 6 abgewandten Seite vorgelagerten zweiten Aufnahmekanal 16 zur Aufnahme mindestens einer zweiten optischen Faser 17 auf. Weiter weist das Messzellengehäuse 5 einen dem dritten Fenster 13 auf seiner dem Messspalt 6 abgewandten Seite vorgelagerten dritten Aufnahmekanal 18 zur Aufnahme mindestens einer dritten optischen Faser 19 auf.

Die Aufnahmekanäle 14, 16, 18 weisen an ihren den Fenstern 11, 12, 13 abgewandten Enden Aufnahmeöffnungen 26, 27, 28 auf, deren Längsachsen 29, 30, 31 parallel zu einander verlaufen.

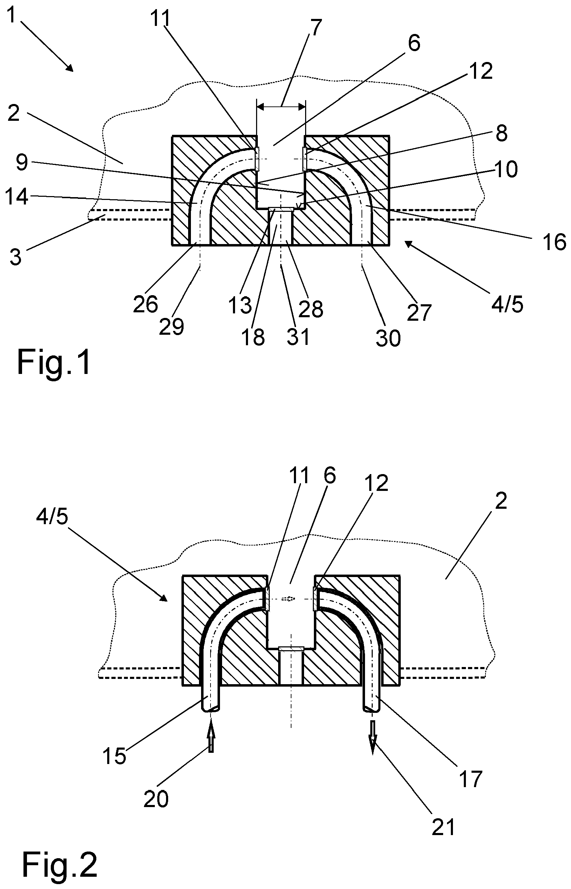

Entsprechend dem Ausführungsbeispiel der Figur 2 ist zur spektroskopischen Transmission Licht 20 einer nicht dargestellten Lichtquelle über die erste optische Faser 15 im ersten Aufnahmekanal 14 durch das erste Fenster 11 in den Messspalt 6 mit dem Probevolumen und weiter über das zweite Fenster 12 und die im zweiten Aufnahmekanal 16 angeordnete zweite Faser 17 als transmittiertes Licht 21 zu einem nicht dargestellten Detektor leitbar. Dabei kann zwischen dem zweiten Fenster 12 und der im zweiten Aufnahmekanal 16 angeordneten zweiten Faser 17 mindestens eine nicht dargestellte Lochblende angeordnet sein.

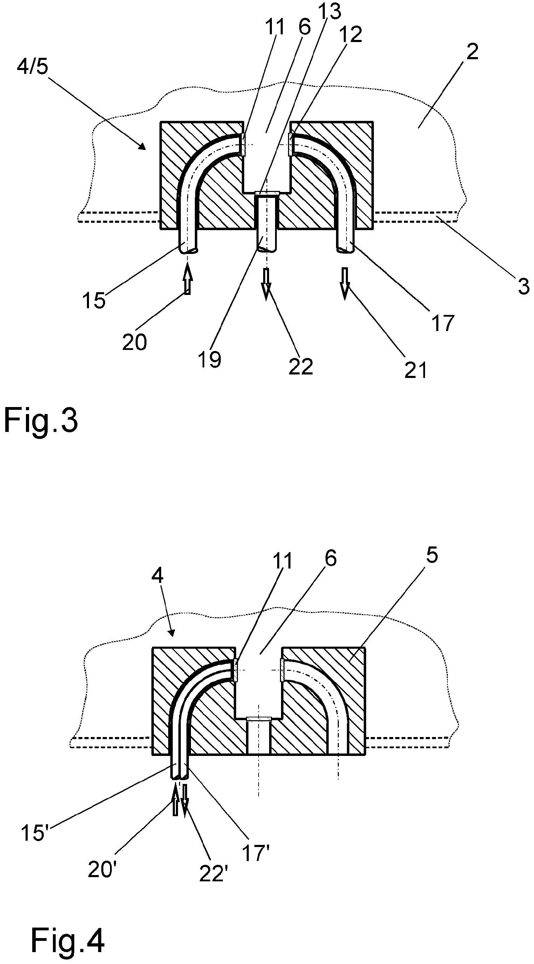

Entsprechend dem Ausführungsbeispiel von Figur 3 ist zusätzlich von dem Probevolumen reflektiertes Licht 22 über das dritte optische Fenster 13 und die im dritten Aufnahmekanal 18 angeordnete dritte optische Faser 19 zu einem nicht dargestellten Detektor leitbar.

Entsprechend dem Ausführungsbeispiel von Figur 4 ist zur Reflektion Licht 20' einer Lichtquelle über eine erste optische Faser 15' im ersten Aufnahmekanal 14 durch das erste Fenster 11 in den Messspalt 6 mit dem Probevolumen leitbar und als vom Probevolumen reflektiertes Licht 22' zurück über das erste Fenster 11 und eine im ersten Aufnahmekanal 14 angeordnete zweite Faser 17' zu einem Detektor leitbar.

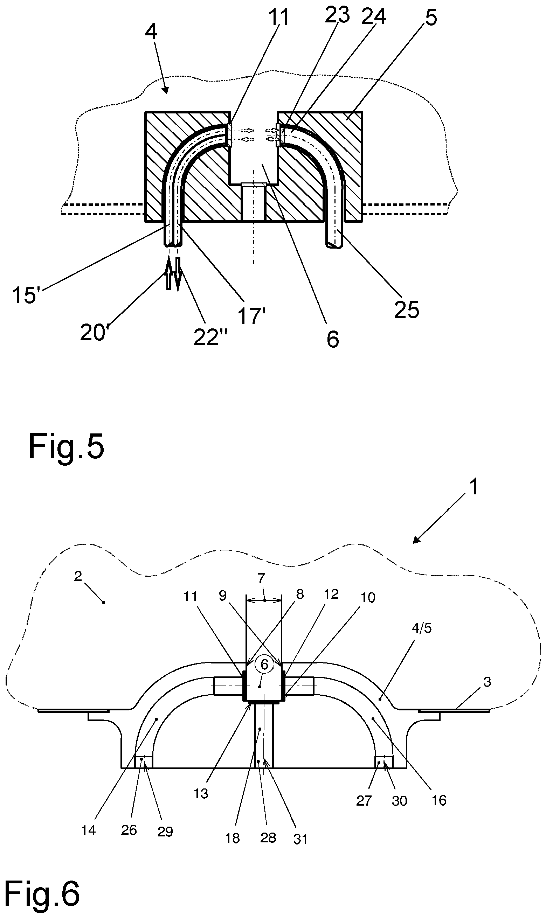

Entsprechend dem Ausführungsbeispiel von Figur 5 ist zur Transflexion Licht 20' einer Lichtquelle über die erste Faser 15' im ersten Aufnahmekanal 14 durch das erste Fenster 11 in den Messspalt 6 mit dem Probevolumen und weiter über das zweite Fenster 12 im zweiten Aufnahmekanal 16 auf eine diffus reflektierende Oberfläche 23 leitbar und ist als reflektiertes Licht 22" zurück über das erste Fenster 11 und eine im ersten Aufnahmekanal 14 angeordnete zweite Faser 17' zu einem Detektor leitbar. Dabei kann die reflektierende Oberfläche 23 am freien Ende einer in den zweiten Aufnahmekanal eingeführten Blindfaser 25 angeordnet sein.

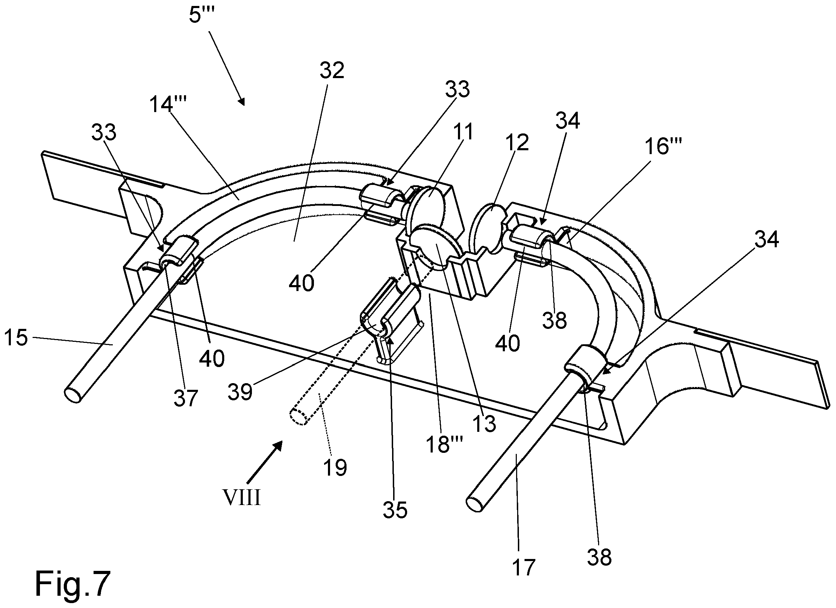

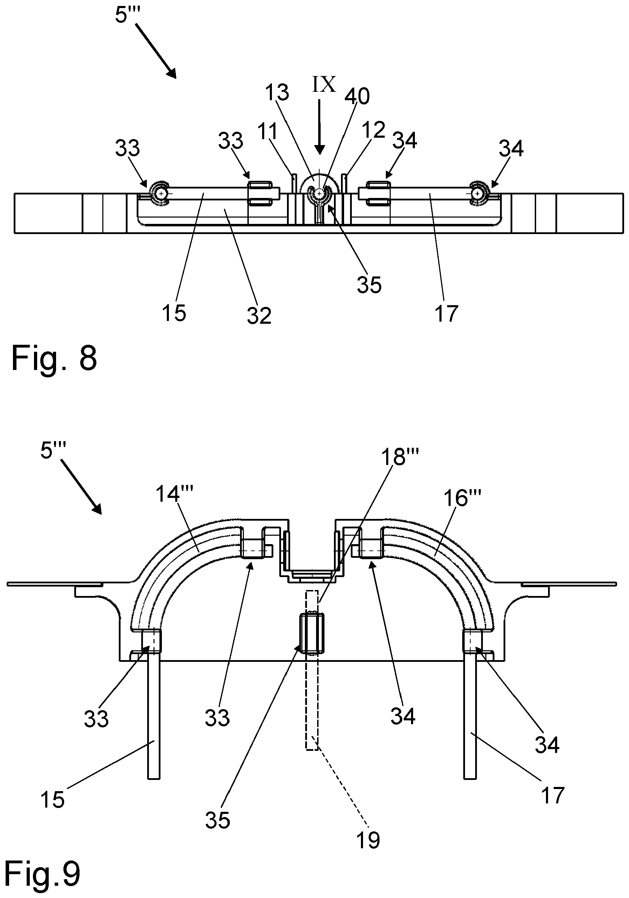

Entsprechend dem Ausführungsbeispiel der Figuren 7 bis 10 bilden die Aufnahmekanäle 14"', 16"', 18'" der Messzelle 5'" eine räumliche Vertiefung 32 in der sie ineinander übergehen. Die Fixierung der optischen Fasern 15, 17, 19 in der Vertiefung 32 erfolgt dabei über Halterungen 33, 34, 35 der der Vertiefung 32 zuzuordnenden Aufnahmekanäle 14'", 16'", 18'". Als Halterungen 33, 34, 35 kommen unter anderem in Frage: Click-Verschlüsse, Bajonett-Verschlüsse und Schraubverschlüsse. In den Halterungen 33, 34, 35 werden die optischen Fasern 15, 17, 19 so fixiert, dass sie mit ihren freien Enden den Fenstern 11, 12, 13 von außen her vorgelagert sind. Die Halterungen 33, 34, 35 weisen jeweils ein Aufnahmekanalstück 37, 38, 39 auf, dessen Aufnahmequerschnitt jeweils ein Kanalsegment zur Aufnahme der zugeordneten optischen Faser 15, 17, 19 bildet. Zur leichteren Fixierung der optischen Fasern 15, 17, 19 können die Aufnahmekanalstücke 37, 38, 39 jeweils einen Längsschlitz 40 aufweisen.

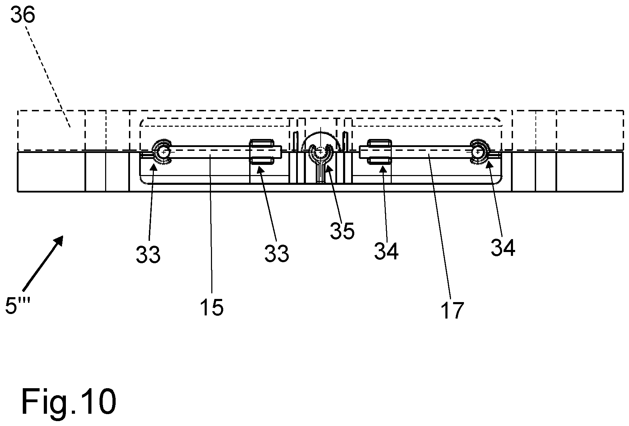

Entsprechend dem Ausführungsbeispiel von Figur 10 wird die Vertiefung 32 der Messzelle 5'" mindestens teilweise durch ein Abdeckteil 36 abgedeckt.

Natürlich stellen die in der speziellen Beschreibung diskutierten und in den Figuren gezeigten Ausführungsformen nur illustrative Ausführungsbeispiele der vorliegenden Erfindung dar. Dem Fachmann ist im Lichte der hiesigen Offenbarung ein breites Spektrum von Variationsmöglichkeiten an die Hand gegeben.

Insbesondere können statt der Fasern 15, 15', 17, 17', 18, 19 auch nicht dargestellte Faserbündel verwendet werden. Dabei können die Fasern 15, 15', 17, 17', 18, 19 oder die Faserbündel vor Gebrauch direkt in die Aufnahmekanäle 14, 16, 18 eingeschoben werden.

Die Fasern 15, 15', 17, 17', 18, 19 oder die Faserbündel können durch Schrauben, Klemmen oder andere nicht dargestellte Fixierungen fest mit dem Messzellengehäuse 5 verbunden werden. Dadurch wird eine reproduzierbare Positionierung der Fasern 15, 15', 17, 17', 18, 19 oder Faserbündel erreicht.

### Bezugszeichenliste

- 1: Behälter
- 2: Behälterinnenraum
- 3: Wandung von 1
- 4: Messzelle
- 5, 5''': Messzellengehäuse von 4
- 6: Messspalt von 5
- 7: Abstand
- 8: erste Seitenfläche
- 9: zweite Seitenfläche
- 10: Verbindungsfläche
- 11: erstes Fenster von 8
- 12: zweites Fenster von 9
- 13: drittes Fenster von 10
- 14, 14''': erster Aufnahmekanal
- 15, 15': erste Faser
- 16, 16''': zweiter Aufnahmekanal
- 17, 17': zweite Faser
- 18, 18''': dritter Aufnahmekanal
- 19: dritte Faser
- 20, 20': Licht
- 21: transmittiertes Licht
- 22, 22', 22": reflektiertes Licht
- 23: diffus reflektierende Oberfläche
- 24: freies Ende von 25
- 25: Blindfaser
- 26: Aufnahmeöffnung
- 27: Aufnahmeöffnung
- 28: Aufnahmeöffnung
- 29: Längsachse
- 30: Längsachse
- 31: Längsachse
- 32: Vertiefung von 5'''
- 33: Halterung von 14'''
- 34: Halterung von 16'''
- 35: Halterung von 18'''
- 36: Abdeckteil
- 37: Kanalstück von 33
- 38: Kanalstück von 34
- 39: Kanalstück von 35
- 40: Längsschlitz von 37, 38, 39

## Patentansprüche

1. Behälter (1) mit einem in seinen Behälterinnenraum (2) hineinragenden Messzellengehäuse (5) einer optischen Messzelle (4), wobei das Messzellengehäuse (5) einen Messspalt (6) aufweist, der von zwei einander in einem Abstand (7) gegenüberliegenden Seitenflächen (8, 9) und einer die Seitenflächen (8, 9) verbindenden Verbindungsfläche (10) begrenzt wird, wobei die Seitenflächen (8, 9) jeweils ein optisches Fenster (11, 12) aufweisen, und wobei dem ersten Fenster (11) mindestens eine erste optische Faser (15, 15') und dem zweiten Fenster (12) mindestens eine zweite optische Faser (17, 17') vorgelagert werden kann,
**dadurch gekennzeichnet,**
**dass** das Messzellengehäuse (5) den Fenstern (11, 12, 13) vorgelagerte Aufnahmekanäle (14, 16, 18) zur Aufnahme der jeweils mindestens einen optischen Faser (15, 15', 17, 17', 19) aufweist,
**dass** die Aufnahmekanäle (14, 16, 18) nachträglich von außen her mit den optischen Fasern (15, 15', 17, 17', 19) bestückbar sind, und
**dass** das Messzellengehäuse (5) mit den Fenstern (11, 12, 13) und Aufnahmekanälen (14, 16, 18) fest mit der Wandung (3) des Behälterinnenraumes (2) verbunden ist.

2. Behälter nach Anspruch 1,
**dadurch gekennzeichnet,**
**dass** die Aufnahmekanäle (14, 14'", 16, 16'", 18'") an ihren den Fenstern (11, 12, 13) zugewandten Enden orthogonal zu den Fenstern (11, 12, 13) ausgerichtet sind.

3. Behälter nach Anspruch 1 oder 2,
**dadurch gekennzeichnet,**
**dass** das Messzellengehäuse (5) an seiner Verbindungsfläche (10) ein drittes optisches Fenster (13) aufweist und
**dass** dem dritten optischen Fenster (13) ein dritter Aufnahmekanal (18) vorgelagert ist, der nachträglich von außen her mit mindestens einer dritten optischen Faser (19) bestückbar ist.

4. Behälter nach einem der Ansprüche 1 bis 3,
**dadurch gekennzeichnet,**
**dass** die Aufnahmekanäle (14, 14"', 16, 16"', 18, 18'") an ihren den Fenstern (11, 12, 13) abgewandten Enden Aufnahmeöffnungen (26, 27, 28) aufweisen deren Längsachsen (29, 30, 31) parallel zu einander verlaufen.

5. Behälter nach einem der Ansprüche 1 bis 4,
**dadurch gekennzeichnet,**
**dass** das Messzellengehäuse (5) mit Fenstern (11, 12, 13) und Aufnahmekanälen (14, 16, 18) einen Single-Use-Teil der optischen Messzelle (4) bildet und
**dass** die optischen Fasern (15, 15', 17, 17', 19) mit verbundenen Lichtquellen und/oder Sensoren und einer Auswerte- und Steuerelektronik einen wiederverwendbaren Teil der optischen Messzelle (4) bilden.

6. Behälter nach einem der Ansprüche 1 bis 5,
**dadurch gekennzeichnet,**
**dass** die optischen Fenster (11, 12, 13) als Linsen ausgebildet sind.

7. Behälter nach einem der Ansprüche 1 bis 6,
**dadurch gekennzeichnet,**
**dass** zur Transmission Licht (20) einer Lichtquelle über die mindestens eine erste Faser (15) im ersten Aufnahmekanal (14, 14'") durch das erste Fenster (11) in den Messspalt (6) mit einem Probevolumen und weiter über das zweite Fenster (12) und die im zweiten Aufnahmekanal (16, 16'") angeordnete mindestens eine zweite Faser (17) zu einem Detektor leitbar ist.

8. Behälter nach Anspruch 7,
**dadurch gekennzeichnet,**
**dass** zwischen dem zweiten Fenster (12) und der im zweiten Aufnahmekanal (16) angeordneten zweiten Faser (17) mindestens eine Lochblende angeordnet ist.

9. Behälter nach einem der Ansprüche 1 bis 6,
**dadurch gekennzeichnet,**
**dass** zur Reflektion Licht (20') einer Lichtquelle über eine erste Faser (15') im ersten Aufnahmekanal (14) durch das erste Fenster (11) in den Messspalt (6) mit einem Probevolumen leitbar und als vom Probevolumen reflektiertes Licht (22') zurück über das erste Fenster (11) und eine im ersten Aufnahmekanal (14) angeordnete zweite Faser (17') zu einem Detektor leitbar ist.

10. Behälter nach einem der Ansprüche 1 bis 6,
**dadurch gekennzeichnet,**
**dass** zur Transflexion Licht (20') einer Lichtquelle über eine erste Faser (15') im ersten Aufnahmekanal (14) durch das erste Fenster (11) in den Messspalt (6) mit einem Probevolumen und weiter über das zweite Fenster (12) im zweiten Aufnahmekanal (16) auf eine diffus reflektierende Oberfläche (23) leitbar ist und als reflektiertes Licht (22") zurück über das erste Fenster (11) und eine im ersten Aufnahmekanal (14) angeordnete zweite Faser (17') zu einem Detektor leitbar ist.

11. Behälter nach Anspruch 10,
**dadurch gekennzeichnet,**
**dass** die reflektierende Oberfläche (23) am freien Ende einer in den zweiten Aufnahmekanal (16) eingeführten Blindfaser (26) angeordnet ist.

12. Behälter nach einem der Ansprüche 3 bis 11,
**dadurch gekennzeichnet,**
**dass** von dem Probevolumen gestreutes Licht (22) über das dritte optische Fenster (13) und die im dritten Aufnahmekanal (18, 18'") angeordnete dritte optische Faser (19) zu einem Detektor leitbar ist.

13. Behälter nach einem der Ansprüche 1 bis 12,
**dadurch gekennzeichnet,**
**dass** der Behälter (1) als ein Einweg-Bioreaktor oder als ein Einweg-Mischbeutel ausgebildet ist.

14. Behälter nach einem der Ansprüche 1 bis 13,
**dadurch gekennzeichnet,**
**dass** die Aufnahmekanäle (14"', 16"', 18'") eine gemeinsame räumliche Vertiefung (32) bilden, in welcher die optischen Fasern (15, 17, 19) durch Halterungen (33, 34, 35) befestigt und den Fenstern (11, 12, 13) vorgelagert sind.

## Claims

1. A container (1) having a measuring-cell housing (5) of an optical measuring cell (4) that protrudes into the container interior (2) of the container, wherein the measuring-cell housing (5) has a measuring gap (6), which is bounded by two opposite-facing lateral surfaces (8, 9) spaced apart from each other at a distance (7) and by a connecting surface (10) that connects the lateral surfaces (8, 9), wherein the lateral surfaces (8, 9) each have an optical window (11, 12), and wherein at least one first optical fiber (15, 15') can be arranged before the first window (11) and at least one second optical fiber (17, 17') can be arranged before the second window (12),
**characterized in that**
the measuring-cell housing (5) has receiving channels (14, 16, 18) arranged before the windows (11, 12, 13) for receiving the at least one optical fiber (15, 15', 17, 17', 19) in each case,
that the receiving channels (14, 16, 18) can be subsequently fitted from the outside with the optical fibers (15, 15', 17, 17', 19), and
that the measuring-cell housing (5) having the windows (11, 12, 13) and the receiving channels (14, 16, 18) is fixedly connected to the wall (3) of the container interior (2).

2. The container according to Claim 1,
**characterized in that**
at their ends facing the windows (11, 12, 13), the receiving channels (14, 14"', 16, 16'", 18'") are oriented orthogonally to the windows (11, 12, 13)

3. The container according to Claim 1 or 2,
**characterized in that**
the measuring-cell housing (5) has a third optical window (13) on its connecting surface (10) and
that before the third optical window (13) there is arranged a third receiving channel (18) that can be fitted from the outside with at least one third optical fiber (19).

4. The container according to any of Claims 1 to 3,
**characterized in that**
at their ends facing away from the windows (11, 12, 13), the receiving channels (14, 14"', 16, 16'", 18, 18'") have receiving openings (26, 27, 28) the longitudinal axes (29, 30, 31) of which extend parallel to one another.

5. The container according to any of Claims 1 to 4,
**characterized in that**
the measuring-cell housing (5) having windows (11, 12, 13) and receiving channels (14, 16, 18) constitutes a single-use part of the optical measuring cell (4) and
that the optical fibers (15, 15', 17, 17', 19), along with connected light sources and/or sensors and evaluation and control electronics, constitute a reusable part of the optical measuring cell (4).

6. The container according to any of Claims 1 to 5,
**characterized in that**
the optical windows (11, 12, 13) are designed as lenses.

7. The container according to any of Claims 1 to 6,
**characterized in that**
for transmission purposes light 20 from a light source can be conducted via the at least one first fiber (15) in the first receiving channel (14, 14'"), through the first window (11) into the measuring gap (6) with a sample volume, and further via the second window (12) and the at least one second fiber (17) arranged in the second receiving channel (16, 16"'), to a detector.

8. The container according to Claim 7,
**characterized in that**
at least one pinhole aperture is arranged between the second window (12) and the second fiber (17) arranged in the second receiving channel (16).

9. The container according to any of Claims 1 to 6,
**characterized in that**
for reflection purposes light 20' from a light source can be conducted via a first fiber (15') in the first receiving channel (14), through the first window (11) into the measuring gap (6) with a sample volume, and can be conducted back as reflected light (22') from the sample volume via the first window (11) and a second fiber (17') arranged in the first receiving channel (14), to a detector.

10. The container according to any of Claims 1 to 6,
**characterized in that**
for transflection purposes light 20' from a light source can be conducted via a first fiber (15') in the first receiving channel (14), through the first window (11) into the measuring gap (6) with a sample volume, and be further conducted via the second window (12) in the second receiving channel (16) onto a diffusely reflecting surface (23), and be conducted back as reflected light (22"),via the first window (11) and a second fiber (17') arranged in the first receiving channel (14), to a detector.

11. The container according to Claim 10,
**characterized in that**
the reflecting surface (23) is arranged at the free end of a dummy fiber (26) inserted into the second receiving channel (16).

12. The container according to any of Claims 3 to 11,
**characterized in that**
scattered light (22) from the sample volume can be conducted via the third optical window (13) and the third optical fiber (19) arranged in the third receiving channel (18, 18"'), to a detector.

13. The container according to any of Claims 1 to 12,
**characterized in that**
the container (1) is designed as a single-use bioreactor or a single-use mixing bag.

14. The container according to any of Claims 1 to 13,
**characterized in that**
the receiving channels (14"', 16"', 18"') form a common spatial depression (32) in which the optical fibers (15, 17, 19) are fastened by means of brackets (33, 34, 35) and are arranged before the windows (11, 12, 13).

## Revendications

1. Récipient (1) présentant un boîtier (5) d'une cellule de mesure optique (4) pénétrant dans l'espace interne (2) du récipient, le boîtier (5) de la cellule de mesure présentant une fente de mesure (6), qui est délimitée par deux surfaces latérales (8, 9) opposées l'une à l'autre à une certaine distance (7) et par une surface de liaison (10) reliant les surfaces latérales (8, 9), les surfaces latérales (8, 9) présentant à chaque fois une fenêtre optique (11, 12), et au moins une première fibre optique (15, 15') pouvant être disposée devant la première fenêtre (11) et au moins une deuxième fibre optique (17, 17') pouvant être disposée devant la deuxième fenêtre (12),
**caractérisé en ce**
**que** le boîtier (5) de la cellule de mesure présente des canaux de réception (14, 16, 18) disposés devant les fenêtres (11, 12, 13) pour recevoir ladite au moins une fibre optique (15, 15', 17, 17', 19) respective,
**que** les canaux de réception (14, 16, 18) peuvent être pourvus ultérieurement, à partir de l'extérieur, des fibres optiques (15, 15', 17, 17', 19),
et **que** le boîtier (5) de la cellule de mesure, conjointement avec les fenêtres (11, 12, 13) et les canaux de réception (14, 16, 18), est relié solidement avec la paroi (3) de l'espace interne (2) de récipient.

2. Récipient selon la revendication 1,
**caractérisé en ce**
**que** les canaux de réception (14, 14"', 16, 16"', 18"') sont alignés orthogonalement par rapport aux fenêtres (11, 12, 13) au niveau de leurs extrémités faisant face aux fenêtres (11, 12, 13).

3. Récipient selon la revendication 1 ou 2,
**caractérisé en ce que**
le boîtier (5) de la cellule de mesure présente une troisième fenêtre optique (13) sur sa surface de liaison (10) et qu'un troisième canal de réception (18) est disposé devant la troisième fenêtre optique (13), lequel peut être pourvu ultérieurement, à partir de l'extérieur, d'au moins une troisième fibre optique (19).

4. Récipient selon l'une quelconque des revendications 1 à 3,
**caractérisé en ce que**
les canaux de réception (14, 14"', 16, 16"', 18, 18"') présentent des ouvertures de réception (26, 27, 28) à leurs extrémités opposées aux fenêtres (11, 12, 13), dont les axes longitudinaux (29, 30, 31) sont parallèles entre eux.

5. Récipient selon l'une quelconque des revendications 1 à 4,
**caractérisé en ce que**
le boîtier (5) de la cellule de mesure forme, avec des fenêtres (11, 12, 13) et des canaux de réception (14, 16, 18), une pièce à usage unique de la cellule de mesure optique (4) et que
les fibres optiques (15, 15', 17, 17', 19) forment, avec des sources de lumière et/ou capteurs reliés et un système électronique d'évaluation et de commande, une pièce réutilisable de la cellule de mesure optique (4).

6. Récipient selon l'une quelconque des revendications 1 à 5,
**caractérisé en ce que**
les fenêtres optiques (11, 12, 13) sont agencées sous forme de lentilles.

7. Récipient selon l'une quelconque des revendications 1 à 6,
**caractérisé en ce que**
pour la transmission, la lumière (20) d'une source de lumière peut être conduite vers un détecteur via la au moins une première fibre (15) dans le premier canal de réception (14, 14"') à travers la première fenêtre (11) dans la fente de mesure (6) comportant un volume d'échantillon, puis, via la deuxième fenêtre (12) et la au moins une deuxième fibre (17) disposée dans le deuxième canal de réception (16, 16"').

8. Récipient selon la revendication 7,
**caractérisé en ce que**
au moins un diaphragme perforé est disposé entre la deuxième fenêtre (12) et la deuxième fibre (17) disposée dans le deuxième canal de réception (16).

9. Récipient selon l'une quelconque des revendications 1 à 6,
**caractérisé en ce que**
pour la réflexion, la lumière (20') d'une source de lumière peut être conduite via une première fibre (15') dans le premier canal de réception (14) à travers la première fenêtre (11) dans la fente de mesure (6) comportant un volume d'échantillon, et peut être reconduite vers un détecteur sous forme de lumière réfléchie (22') par le volume d'échantillon via la première fenêtre (11) et une deuxième fibre (17') disposée dans le premier canal de réception (14).

10. Récipient selon l'une quelconque des revendications 1 à 6,
**caractérisé en ce que**
pour la transflexion, la lumière (20') d'une source de lumière peut être conduite via une première fibre (15') dans le premier canal de réception (14) à travers la première fenêtre (11) dans la fente de mesure (6) comportant un volume d'échantillon, puis, via la deuxième fenêtre (12) dans le deuxième canal de réception (16), sur une surface réfléchissante (23) de manière diffuse, et peut être reconduite vers un détecteur sous forme de lumière réfléchie (22") via la première fenêtre (11) et une deuxième fibre (17') disposée dans le premier canal de réception (14).

11. Récipient selon la revendication 10,
**caractérisé en ce que**
la surface réfléchissante (23) est disposée à l'extrémité libre d'une fibre aveugle (26) insérée dans le deuxième canal de réception (16).

12. Récipient selon l'une quelconque des revendications 3 à 11,
**caractérisé en ce que**
la lumière (22) diffusée par le volume d'échantillon peut être conduite vers un détecteur via la troisième fenêtre optique (13) et la troisième fibre optique (19) disposée dans le troisième canal de réception (18, 18"').

13. Récipient selon l'une quelconque des revendications 1 à 12,
**caractérisé en ce que**
le récipient (1) est conçu comme bioréacteur jetable ou comme sac de mélange jetable.

14. Récipient selon l'une quelconque des revendications 1 à 13,
**caractérisé en ce que**
les canaux de réception (14'", 16'", 18"') forment une dépression spatiale commune (32), dans laquelle les fibres optiques (15, 17, 19) sont fixées par des fixations (33, 34, 35) et sont disposées devant les fenêtres (11, 12, 13).
